# EUROPEAN PATENT APPLICATION

(11) **EP 4 353 156 A1**
(43) Date of publication of application: **17.04.2024**
(21) Application number: 22200619.9
(22) Date of filing: 10.10.2022
(51) Int. Cl.: A61B 8/08, A61B 8/14, A61B 8/00

(54) **ULTRASOUND MEASUREMENT SYSTEM AND METHOD**

(71) Applicant: UTC Imaging B.V., 6171 GD Stein (NL)
(72) Inventor: BAKKER, Erwin Maria, 6171 GD Stein (NL); VAN SCHIE, Johannes Theodorus Maria, 6171 GD Stein (NL)
(74) Representative: DeltaPatents B.V.

(57) **Abstract**

A system is provided comprising a B-mode ultrasound transducer and a support structure for being placed on or near a human or animal body, wherein the support structure comprises a holding device for the ultrasound transducer which is movable and tiltable about the short axis of the ultrasound transducer. The system is configured to move the ultrasound transducer in a first pass along a spatial axis to acquire a first series of images at a respective series of positions, move the ultrasound transducer in a second pass along the spatial axis to acquire a second series of images at the series of positions, and tilt the holding device to have the ultrasound transducer assume first tilt angle during the first pass and a second tilt angle during the second pass. By imaging a same anatomical part using different tilting angles, it is easier to visualize 3D ultrastructure, to characterize and quantify 3D ultrastructure by means of measuring the amount of anisotropy in tissue, and/or to distinguish structure-related reflections from random scattering.

## Description

### FIELD OF THE INVENTION

The invention relates to an ultrasound measurement system and to a computer-implemented method of ultrasound imaging for use with the ultrasound measurement system. The invention further relates to a computer-readable medium comprising data representing a computer program, wherein the computer program comprises instructions for causing a processor system to perform the method.

### BACKGROUND ART

Ultrasound imaging has been used for many decennia as a non-invasive tool for visualization of biological and organic tissues such as ligaments, muscles, and tendons. Such visualizations may be generated for various purposes, including but not limited to medical diagnosis, medical studies, and therapy implementations.

B-mode ultrasound imaging, which is also known as 2D mode imaging, involves using a transducer containing a linear array of elements to simultaneously scan a plane through the body that can be viewed as a two-dimensional image on-screen. Typically, in B-mode ultrasound imaging, a probe is used, which probe may itself also be referred to as an 'ultrasound transducer'. If the probe is handheld, which it often is, the probe is subject to six Degrees of Freedom (DoF). Disadvantageously, the six DoF result in limited reproducibility of the acquired ultrasound images, as it is difficult to find the exact area of interest back. In addition, qualitative assessment, and quantitative grayscale statistics of the acquired 2D ultrasound images may not be representative of 3D structures. Another drawback of B-mode ultrasound imaging is that in echo patterns obtained by a B-mode ultrasound transducer, refractions and especially random scattering may interfere with structure-related reflections. This may make it more difficult for a user or an image analysis algorithm to correctly characterize and quantify the ultrastructure of insonated tissues from ultrasound images acquired by B-mode ultrasound imaging.

3D ultrasound imaging addresses at least some of the disadvantages of 2D ultrasound imaging since in 3D ultrasound imaging the DoF are typically constrained (e.g., by movement along one or more spatial axes being actuated and therefore controlled) and/or by at least some of the DoF being tracked. An example of such a 3D ultrasound imaging system is described in a publication by Plevin S, McLellan J, van Schie H, Parkin T. [1], which describes a system in which an ultrasound transducer is mounted in a motorized tracking-device and transversal images are acquired at regular distances. It is said that conventional ultrasonography is not sufficiently sensitive to accurately monitor tendon and predict injury, and refers to, and makes use of Ultrasound tissue characterisation (UTC), a relatively new technique, which improves tendon characterisation by providing a 3-dimensional (3D) SDFT reconstruction and objective calculation of fibre alignment by classifying fibres into one of 4 echo-types.

It is desirable to further improve upon known ultrasound imaging techniques. For example, both 2D and 3D ultrasound imaging suffer from the problem that it is difficult to quantitatively assess the amount of anisotropy in tissues. Also, known 3D ultrasound imaging techniques continue to suffer from refractions and especially random scattering interfering with structure-related reflections.

### References

[1] Plevin S, McLellan J, van Schie H, Parkin T. Ultrasound tissue characterisation of the superficial digital flexor tendons in juvenile Thoroughbred racehorses during early race training. Equine Vet J. 2019 May;51(3):349-355. doi: 10.1111/evj.13006. Epub 2018 Sep 18. PMID: 30125384.

### SUMMARY OF THE INVENTION

A first aspect of the invention provides an ultrasound measurement system which comprises:
- an ultrasound transducer, wherein the ultrasound transducer is a B-mode ultrasound transducer for imaging a part of an anatomical structure;
- a support structure for being placed on or near a human or animal body which comprises the anatomical structure, wherein the support structure comprises a holding device for the ultrasound transducer, wherein the holding device is movable along a spatial axis in physical space, wherein the holding device is oriented so that a short axis of the ultrasound transducer is orthogonal to the spatial axis, and wherein the holding device is tiltable about the short axis of the ultrasound transducer;
- an actuation subsystem for moving the holding device and thereby the ultrasound transducer along the spatial axis and for tilting the holding device and thereby the ultrasound transducer;
- a processing subsystem configured to, using the actuation subsystem and the ultrasound transducer:
   - move the ultrasound transducer in a first pass along the spatial axis to acquire a first series of images at a respective series of positions along the spatial axis, move the ultrasound transducer in a second pass along the spatial axis to acquire a second series of images at the series of positions along the spatial axis, and before each pass, tilt the holding device to have the ultrasound transducer assume a first tilt angle during the first pass and a second tilt angle during the second pass;
   - generate a visualization of the part of the anatomical structure based on the first series of images and the second series of images.

A further aspect of the invention provides a computer-implemented method of ultrasound imaging for use with the ultrasound measurement system as described in this specification. The computer-implemented method comprises, at the processing subsystem of the ultrasound measurement system:
- controlling the actuation subsystem and the ultrasound transducer to move the ultrasound transducer in a first pass along a spatial axis in physical space and to acquire a first series of images at a respective series of positions along the spatial axis;
- controlling the actuation subsystem and the ultrasound transducer to move the ultrasound transducer in a second pass along the spatial axis and to acquire a second series of images at the series of positions along the spatial axis;
- controlling the actuation subsystem to, before each pass, tilt the holding device to have the ultrasound transducer assume a first tilt angle during the first pass and a second tilt angle during the second pass; and
- generating a visualization of the part of the anatomical structure based on the first series of images and the second series of images.

A further aspect of the invention provides a transitory or non-transitory computer-readable medium comprising data representing a computer program, the computer program comprising instructions for causing a processor system to perform the computer-implemented method as described in this specification.

The above measures involve using an ultrasound transducer to acquire images of a part of an anatomical structure of a human or animal body. The part of the anatomical structure may for example be a tissue part. The ultrasound transducer may be a B-model ultrasound transducer as described in the background section of this specification and may elsewhere also be referred to in short as 'ultrasound transducer' or simply as 'probe'. The ultrasound transducer may, but does not need to be, a transducer which is capable of being used by hand, i.e., a handheld type of ultrasound transducer. In the ultrasound measurement system, however, the ultrasound transducer is held by a support structure which allows the ultrasound transducer to be brought into the vicinity of a human or animal body. For example, the support structure may be placed on the human or animal body, and therewith, the ultrasound transducer may be brought into contact with the skin of the body, or at least in close vicinity of the skin. Such contact may be direct contact or indirect contact, e.g., via acoustic coupling media.

More specifically, the ultrasound transducer may be held by a holding device which may be part of the support structure, e.g., a component thereof. The holding device may be movable along a spatial axis in physical space, that is, along a substantially straight line. For example, the holding device may be slidable along one or more guide rails which are substantially straight. The movement of the holding device may be effected by an actuation subsystem of the ultrasound measurement system. For example, the actuation subsystem may comprise one or more linear actuators or linear stepper motors so as to move the holding device. Such movement may, but does not need to be, tracked by measurements, for example by using a closed loop control system to track the movement. In other examples, the linear actuators may be sufficiently accurate to avoid the need for tracking using measurements. In such examples, the control data send to the linear actuators may be used as basis to derive positional information, e.g., to determine at which positions along the spatial axis the images are acquired. In addition to being moveable, the holding device may be tiltable, with the tilting axis corresponding to the short axis of the ultrasound transducer when the ultrasound transducer is held by the holding device. In other words, the ultrasound transducer may be tilted around the contact line of the ultrasound transducer with the subject, as also shown in the various figures. The tilting may also be actuatable by the actuation subsystem, e.g., using motor(s).

The ultrasound measurement subsystem may further comprise a processing subsystem which may be configured to control the actuation subsystem and thereby control the movement and tilting of the holding device including the ultrasound transducer. The processing subsystem may for example be a computer, such as desktop or a laptop, or a combination of a computer and one or more microcontrollers, etc., and may be configured by software to perform certain actions. In particular, the processing subsystem may be configured to acquire at least two series of images of the part of the anatomical structure. Each series of images may be acquired by the ultrasound transducer being moved in a respective pass along the spatial axis, and thereby over the surface of the body. The images in each series may be acquired at a series of respective positions along the spatial axis. Each series of images may be considered to represent a separate 3D ultrasound data set showing the part of the anatomical structure. To acquire the series of images, the processing subsystem may be configured to control and receive ultrasound data from the ultrasound transducer.

In accordance with the above measures, the ultrasound transducer may be tilted differently during each pass. For example, the ultrasound transducer may be tilted +15 degrees with respect to a surface normal of the body in one pass and -15 degrees in another pass. For that purpose, when acquiring a series of images, the ultrasound transducer may be moved in a controlled way along one spatial axis while the other five DoF of the ultrasound transducer are constrained, which include the ultrasound transducer being tilted to assume a different yet known value in each of the passes. This way, at least two series of images of the anatomical structure may be acquired at substantially the same positions along the spatial axis but with the ultrasound transducer being tilted differently for each series of images. A visualization of the part of the anatomical structure may then be generated using the two or more acquired 3D ultrasound data sets, for example by combining them to obtain a single 3D ultrasound data set which may be visualized in various ways, e.g., by showing several cross-sectional views of the 3D ultrasound data set or by so-called volume rendering.

It has been found that by constraining the DoF of the ultrasound transducer, e.g., by keeping five DoF fixed during the movement and moving the ultrasound transducer along the sixth DoF, the measurement is repeatable, and in fact, repeatable over different passes. This allows 3D ultrasound data sets to be acquired in which (only) the tilt angle is different while other parameters of the scan are kept the same.

Moreover, by having two or more 3D ultrasound data sets acquired using different tilt angles, at least some problem(s) of prior art ultrasound imaging techniques may be addressed. Namely, the image acquisition at different tilt angles may provide extra information which may enable a viewer of the ultrasound images, or an image processing algorithm or model, to better distinguish between on the one hand reflections which have a direct, or 1-to-1, relationship with an anatomical structure, such as an interface between different tissue, and on the other hand echo patterns caused by refractions and scattering. The latter type of echo patterns may not have a 1-to-1 relationship with an anatomical structure. This may be explained as follows.

There are different types of tissues. These tissues, as well as the transitions (interfaces) between them, generate an echo pattern that is the resultant of various physical interactions, including reflections (specular or diffuse), refractions and scattering. Reflections may generate echoes that have a 1-to-1 relationship with the insonated interface and are therefore also referred to as being 'structure-related' echoes. In contrast, refractions and scattering may occur randomly and lack a clear 1-to-1 relationship with the insonated interface. As such, their resulting echoes are not considered to be directly related to a particular structure. The extent to which these different physical interactions occur may be determined by the differences in acoustic impedance ("density") and the 3D ultrastructure of the tissues and in particular the arrangement and size of the interfaces. Depending on the 3D ultrastructure of different tissue types, the echo pattern may be generated to a greater or lesser extent by reflections, being either specular or diffuse. These may be indistinguishable in a 2D ultrasound image from randomly occurring refractions and scattering. In addition, specular reflections from a tight transition (interface) and diffuse reflections from an erratic transition (interface) may be difficult to distinguish from each other in a 2D ultrasound image. These reflections may differ for different tissues and transitions, and accordingly, the type of reflection may be indicative of the type of tissue/transition, e.g., physiological versus pathological.

By insonating the tissues using different tilt angles, which may also be referred to as multi-angle insonation, more information is obtained than when using only a single insonation angle, which in turn allows to specular reflections, diffuse reflections, scattering and refraction to be better distinguished from each other since these phenomena are at least in part angle dependent. In particular, by taking into account how the echo pattern varies (or does not vary) as function of angle, one may therefore better determine which type of tissue/transition is being shown in the acquired ultrasound image data. By way of the above measures, for a given voxel location, ultrasound data may be available from two or more tilt angles. For example, consider that N passes are performed at different tilt angles. As a result, N intensity values (intensity of echoes, expressed as their brightness or grey level) may be available for each voxel location (with each voxel location corresponding to a physical location in the human or animal body). The N intensity values may form a *N*-dimensional feature vector, which may be further processed. Such a feature vector may be used for multidimensional analysis to determine whether the echo pattern is structure-related or not, for 3D visualization of interfaces and may allow for a characterization of the ultrastructure of different tissue types.

The following optional aspects of the invention may be described with reference to the ultrasound measurement system but equally apply to the computer-implemented method and the computer program as described in this specification.

Optionally, the first tilt angle and the second tilt angle differ in sign with respect to a neutral tilt angle. Optionally, the first tilt angle and the second tilt angle are substantially identical in magnitude. This way, at least some of the tilt angles are mirror-symmetric with respect to the surface normal, e.g., +X and -X degrees.

Optionally, the processing subsystem is configured to acquire three or more series of images in three or more passes using three or more different tilt angles.

Optionally, the three or more different tilt angles include a neutral tilt angle.

Optionally, each respective tilt angle is selected within a range of -20 to 20 degrees, preferably within a range of -15 to 15 degrees. These ranges allow a wide range of observation of the part of the anatomical structure to be imaged.

Optionally, the series of positions along the spatial axis is a series of equidistant positions, for example arranged at respective distances selected within a range of 0.05 mm to 0.5 mm, preferably within a range of 0.1 mm to 0.3 mm. For example, a step-size of 0.2 mm may be used, with the term 'step-size' referring to the distance between consecutive images. In general, the distance or step-size may be selected based on the elevation (or azimuthal dimension) of the beam of the ultrasound transducer, i.e., the focus, the number of focal points, the focal range (e.g., narrow vs. widespread), etc. In general, smaller step sizes may be preferred for increase in spatial resolution.

Optionally, the processing subsystem is configured to move the ultrasound transducer in consecutive passes in alternating direction along the spatial axis. This way, it is not needed for the ultrasound transducer to be moved back into the same starting position after the first scan. Rather, the second scan may start at the end position of the first scan, which may avoid unnecessary movement of the ultrasound transducer and may result in a shorter image acquisition time and faster examination.

Optionally, the processing subsystem is configured to generate the visualization by:
- reconstructing a 3D volume showing the imaged part of the anatomical structure based on the first series of images and the second series of images;
- visualizing the 3D volume, for example by generating 2D images representing intersections of the 3D volume along sagittal, coronal, and transversal planes and by visualizing the 2D images.

The ultrasound data sets obtained at the respective tilt angles may be combined into a single 3D volume which may then be visualized to the user, for example as cross-sectional views of the 3D ultrasound data set or by volume rendering. Such a 3D volume may facilitate the interpretation of the image data of all 3D ultrasound data sets since intensity values corresponding to a same physical location in the human or animal body are mapped to a same voxel in the 3D volume.

Optionally, the processing subsystem is configured to generate the 3D volume using a 3D reconstruction technique and using the respective tilt angles of the ultrasound transducer as parameters in the 3D reconstruction technique. The 3D reconstruction technique may for example be an image rectification technique which uses the tilt angles to determine the angle of projection of the acquired images onto a common plane or into a common volume having a common coordinate system.

Optionally, in the 3D reconstruction technique, reconstructed intensities from different series of images are assigned to different colour components of the 3D volume. This way, visual information obtained from the separate image series is maintained and may be viewed separately or easily distinguished from each other.

Optionally, the processing subsystem is further configured to:
- extract features pertaining to the part of the anatomical structure from corresponding parts of the first series of images and the second series of images;
- classify and/or segment the part of the anatomical structure using said extracted features.

Optionally, the processing subsystem is configured to classify and/or segment the part of the anatomical structure by using the extracted features as input to a machine learned model, such as a deep neural network, rule-based classifiers, decision trees, random forests, gradient boosting machines, etc.

Optionally, the part of the anatomical structure is a tissue part.

It will be appreciated by those skilled in the art that two or more of the above-mentioned embodiments, implementations, and/or aspects of the invention may be combined in any way deemed useful.

Modifications and variations of the computer-implemented method and/or the computer program, which correspond to the described modifications and variations of the ultrasound measurement system, or vice versa, can be carried out by a person skilled in the art on the basis of the present description.

### BRIEF DESCRIPTION OF THE DRAWINGS

These and other aspects of the invention will be apparent from and elucidated further with reference to the embodiments described by way of example in the following description and with reference to the accompanying drawings, in which
Figs. 1A-1D illustrate physical interactions between incident ultrasound waves and the different interfaces in the insonated matrix;
Fig. 2 schematically shows an ultrasound measurement system which is configured to move an ultrasound transducer in different passes over a part of an anatomical structure of a subject, and in which passes the ultrasound transducer assumes different tilt angles relative to a surface normal of the exterior of the subject;
Figs. 3A-3D illustrate the ultrasound transducer assuming different tilt angles during different passes over the anatomical structure;
Fig. 4A shows a support structure which is configured to hold the ultrasound transducer, and which support structure comprises guide rails and a motorized carriage configured to move the ultrasound transducer along a spatial axis;
Fig. 4B shows the tilting of the ultrasound transducer;
Fig. 4C shows a cutaway view of the support structure, showing internal guide rails and a motorized carriage configured to move along the guide rails;
Fig. 5 shows the processing subsystem of the ultrasound measurement system, which processing subsystem comprises an ultrasound transducer interface to the ultrasound transducer and an actuator interface to the actuation subsystem; and Fig. 6 shows a computer-readable medium comprising data.

It should be noted that the figures are purely diagrammatic and not drawn to scale. In the figures, elements which correspond to elements already described may have the same reference numerals.

### List of reference numbers and abbreviations

The following list of reference numbers is provided for facilitating the interpretation of the drawings and shall not be construed as limiting the claims.
- 1: incident ultrasound wave
- 2: interface in insonated matrix

- 20: subject
- 22: surface normal
- 40: ultrasound signal
- 42: angle relative to surface normal
- 60: linear displacement along spatial axis
- 80: short axis
- 82: tilting about short axis
- 90: display
- 92: user input device

- 100: ultrasound measurement system
- 200: B-mode ultrasound transducer
- 210: acquisitions at tilting angle
- 220: series of acquisitions at first tilting angle
- 222: series of acquisitions at second tilting angle
- 224: series of acquisitions at third tilting angle

- 300: support structure
- 305: recess for receiving body part
- 310: holding device
- 320: housing
- 330: guide rails
- 340: motorized carriage

- 400: processing subsystem
- 410: processor(s)
- 420: actuator interface
- 430: ultrasound transducer interface
- 440: user interface subsystem

- 500: computer-readable medium
- 510: stored data

### DETAILED DESCRIPTION OF EMBODIMENTS

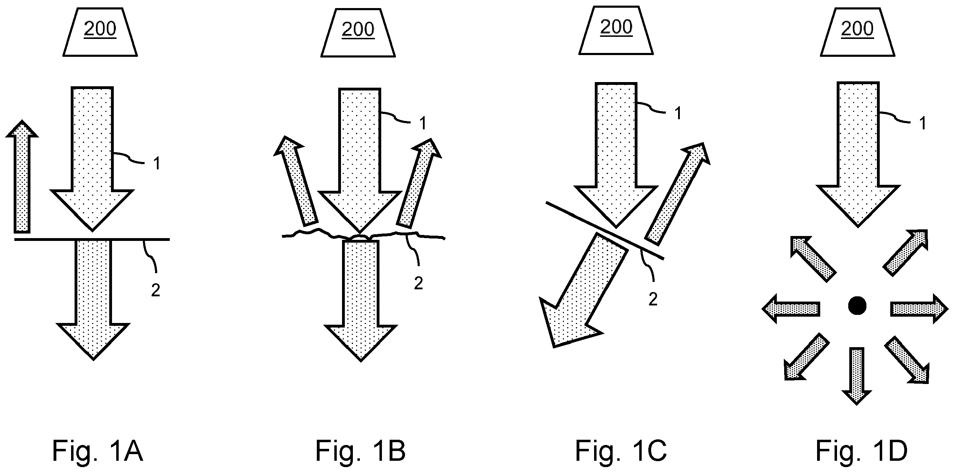

**Figs. 1A-1D** show different physical interactions between incident ultrasound waves 1 which are transmitted by an ultrasound transducer 200 in form of an ultrasound pulse and one or more interfaces 2 within the human or animal body. Such an interface may for example be an interface within the extracellular matrix, e.g., between different tissue types, and may represent a physical interface within the human or animal body as well as an acoustic interface for the ultrasound waves 1.

The echo-patterns which are obtained during ultrasound measurements may be the result of such physical interactions between the incident ultrasound waves 1 and the interface(s) 2, and may be affected by a number of factors, which factors include, but are not limited to, the wavelength of the ultrasound waves, differences in the acoustic impedance at the interface(s), the angle of insonation at interface(s), the arrangement and integrity of the interface(s) and the size and separation of the interface(s). When assessing ultrasound images, e.g., for diagnostic or treatment purposes, criteria for such assessment include, but are not limited to:
- reflectivity, intensity of echoes
- steadiness of individual echoes
- anisotropy, which is the property of for example tendons, muscles, and nerves to vary their ultrasound appearance depending on the angle of insonation of the incident ultrasound beam. Anisotropy is not to be mistaken for existing disintegration (pathology). Anisotropy may for example occur when the incident ultrasound waves are oblique to acoustic interfaces, thereby causing a hypoechoic artifact in the echo.

In general, there may be different physical interactions at an interface, e.g.:
a) Specular reflection, as illustrated in Fig. 1A.
b) Diffuse reflection, as illustrated in Fig. 1B.
c) Refraction, as illustrated in Fig. 1C.
d) Scattering, as illustrated in Fig. 1D.

Specular reflections as illustrated in Fig. 1A may occur at interfaces that are intact (e.g., are regular, have a sharp outline), have a reflector separation which is larger than the ultrasound wavelength, and that are perpendicular to the incident ultrasound waves. When assessing ultrasound images, specular reflections may be identified on the basis of one or more of the following characteristics:
- there is a direct (one-one) relationship between the ultrasound pulse, the generated echo, and relatively large structures in the matrix, such as, but not limited to, uniaxially arranged fascicles in tendons, ligaments, and muscles. These reflections generate "structure-related" echoes.
- highly reflective when insonated perpendicularly.
- steady over contiguous pixels or voxels while moving the ultrasound transducer along a spatial axis.

Diffuse reflections as illustrated in Fig. 1B may occur at interfaces that are, in contrast to the interfaces causing specular reflections, less sharp, more irregular and erratic, causing the reflections to become dispersed, with the interfaces having a reflector separation which exceeds the ultrasound wavelength such as, but not exclusively, fascicles in tendons, ligaments and muscles. When assessing ultrasound images, diffuse reflections may be identified on the basis of one or more of the following characteristics:
- although the reflector separation exceeds the ultrasound wavelength and thus the size of the structures is above the limits of resolution, the direct (one-one) relationship between echo and structure may be moderate or even weak. This may for example occur when fascicles are swollen and/or surrounding layer of loose connective tissue (e.g., endotenon) is irregularly thickened, indicative for pathology.
- moderately reflective when insonated perpendicularly.
- moderate steadiness over contiguous pixels or voxels when moving the ultrasound transducer along a spatial axis.

Refraction as illustrated in Fig. 1C occurs at interfaces of structures having a size which exceeds the ultrasound wavelength, however, only when these interfaces are not insonated perpendicularly, leading to loss of echogenicity as a consequence of dispersion of reflections. When assessing ultrasound images, refraction areas may be erroneously identified as substantial loss of structure-related reflectors, e.g., hypo-echogenicity which is artefactual when these reflectors are present but not insonated perpendicularly which is a physiological phenomenon. However, hypo-echogenicity may also be the result of the absence of structure-related reflectors with a size exceeding the ultrasound wavelength, and may thus also be indicative for disintegration (pathology). When assessing ultrasound images, refraction may be identified on the basis of one or more of the following characteristics:
- there is no reflectivity at all or otherwise echoes are lacking any direct (one-one) structural relationship due to the fact that structures are not insonated perpendicularly.
- when insonated under different angle reflectivity may increase due to the fact that structures with size above ultrasound wavelength may become insonated perpendicularly, thus becoming structure-related reflectors.
- substantial variation of reflectivity over contiguous pixels or voxels while moving the ultrasound transducer along a spatial axis.
- substantial variation of reflectivity over contiguous pixels or voxels while changing angle of insonation.

Scattering as illustrated in Fig. 1D may occur when an ultrasound pulse hits multiple (very) small interfaces having a separation which is (much) smaller than the ultrasound wavelength. When assessing ultrasound images, scattering may be identified on the basis of one or more of the following characteristics:
- existence of multiple interfering echoes that may spread in all directions.
- mostly low, sometimes moderate, intensity of echoes (echogenicity) depending on the acoustic impedance, which may be related to density of collagenous matrix.
- lack of steadiness over contiguous in pixels and voxels while moving the ultrasound transducer along a spatial axis.
- barely any variation of intensity while changing angle of insonation.

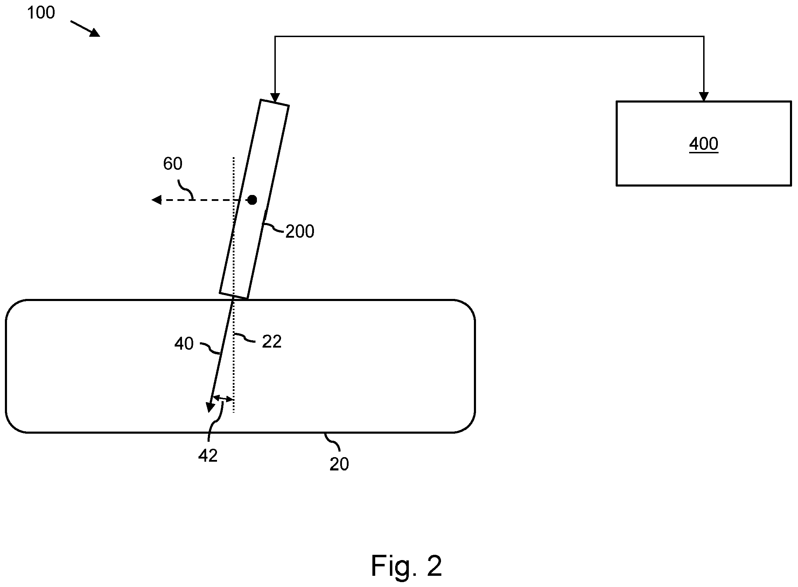

Fig. 2 schematically shows select parts of an ultrasound measurement system 100. For sake of illustration, some parts of the ultrasound measurement system 100 are omitted in Fig. 2 but are described elsewhere in this specification. Generally, speaking, the ultrasound measurement 100 may comprise a B-mode ultrasound transducer which is shown schematically in Fig. 2 as an elongated object 200. The ultrasound transducer 200 may be held by a support structure (not shown in Fig. 2) and may be movable along a spatial axis 60 in physical space, by which the ultrasound transducer may be moved over the surface (e.g., skin) of a human or animal body 20. As is known per se, the ultrasound transducer may emit ultrasound signals 40 and may receive reflections of the emitted ultrasound signals. These reflections may be sent as data to a processing subsystem 400 of the ultrasound measurement system 100, where they may be stored, further processed and/or visualized on a display.

With continued reference to Fig. 2, the ultrasound transducer 200 may be tiltable about its short axis. The tilting may be actuated, which actuation may be controlled by the processing subsystem 400. Accordingly, the processing subsystem 400 may cause the ultrasound transducer to assume a tilt angle 42 relative to a surface normal 22 of the surface of the body 20 (or relative to an axis of the support structure which is meant to be parallel to said body surface when the support structure is placed on the body 20). As such, the ultrasound transducer 200 may be moved over the surface of the body 20 while being tilted with respect to the surface normal 22. The processing subsystem 400 may during such movement acquire a series of images, with each of the image being acquired at a different position along the spatial axis 60.

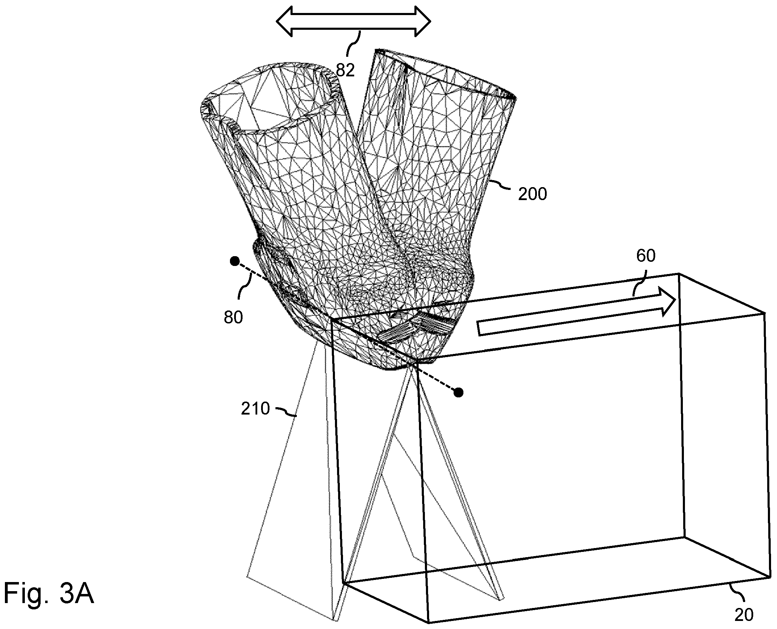

**Figs. 3A-3D** provide further illustration of the ultrasound transducer assuming different tilt angles during different passes over the anatomical structure. In these figures, only the ultrasound transducer 200 is shown while other parts of the ultrasound measurement system, for example the support structure, the actuation subsystem, or the processing subsystem, are omitted for sake of illustration. Moreover, the body 20 to be imaged is shown in Figs. 3A-3D simply as a transparent box.

**Fig. 3A** shows the ultrasound transducer 200 being placed on the subject's body 20 so as to acquire one or more images of the interior of the body 20. While the images are themselves are not shown in Fig. 3A, the corresponding imaging planes 210 are shown at which the images are acquired, and which imaging planes may symbolize the acquired images. Fig. 3A further shows that the ultrasound transducer 200 can be tilted about the short axis 80 of the ultrasound transducer, with the tilting being schematically indicated by a bidirectional arrow 82 showing that the tilting may be in both directions, e.g., forward and backward, which may correspond to a negative tilt angle and a positive tilt angle, or vice versa depending on how the sign of the tilt angle is defined. The ultrasound transducer 200 may thus be placed on the subject's body 20 with pan and roll both set to 0 degrees and with the tilt angle being selectable, e.g., from a range of tilt angles. Fig. 3A further shows the ultrasound transducer being moveable over the body along a spatial axis 60 while its position along the other two orthogonal axis is maintained. For example, if the spatial axis 60 is considered as the x-axis, the ultrasound transducer 200 may be kept at the same y- and z-coordinates.

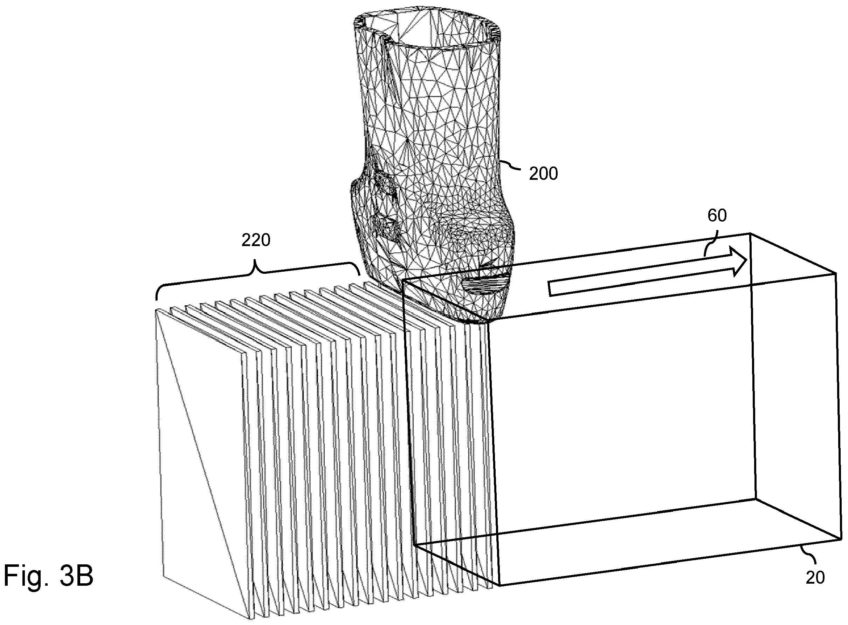

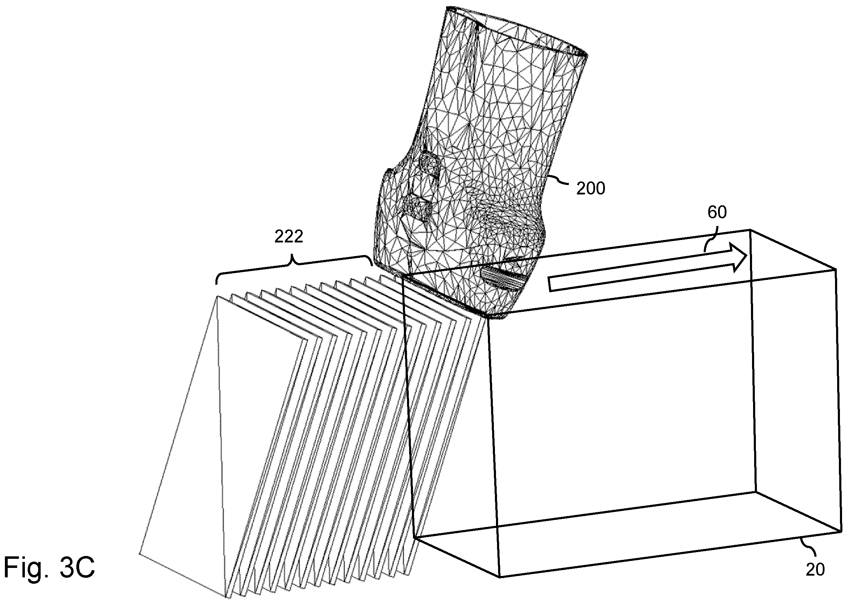

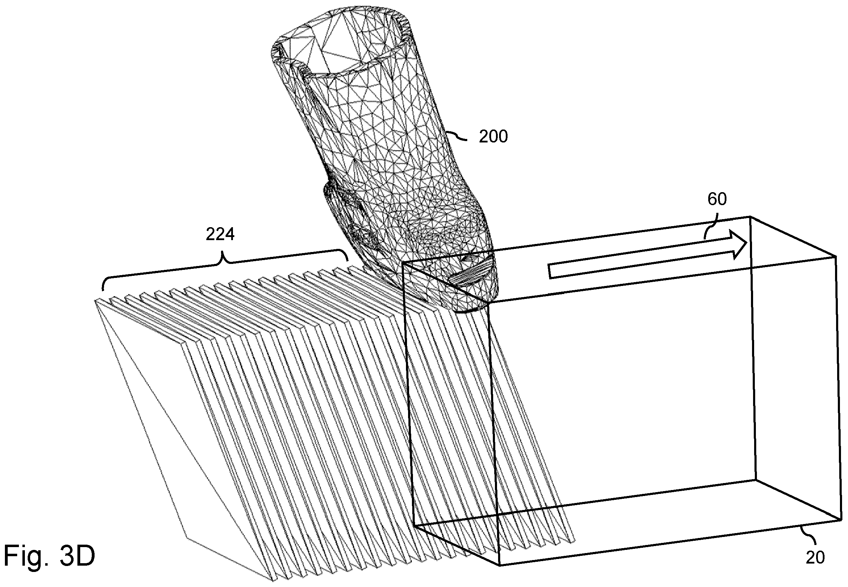

**Fig. 3B** illustrates the acquisition of a first series 220 of images by the ultrasound transducer 200. In this example, the ultrasound transducer 200 is moved along the designated spatial axis 60 while at equidistant positions along the spatial axis images are acquired and stored. As shown in Fig. 3B, this first series 220 of images may be acquired while the ultrasound transducer is at a neutral tilt angle, which may refer to the tilt angle with respect to the body's surface normal being substantially zero. **Fig. 3C** shows a second series 222 of images being acquired by the ultrasound transducer 200. This second series 222 may be acquired at the same respective positions along the spatial axis 60 as the first series of images 200 but with a different tilt angle of the ultrasound transducer 200. **Fig. 3D** shows a third series 224 of images being acquired by the ultrasound transducer 200. This third series 224 of images may again be acquired at the same respective positions along the spatial axis 60 as the first series 220 and the second series 222 of images, and again at a different tilt angle.

As will be further explained elsewhere, the image acquisitions shown in Figs. 3A-3D may be controlled by the processing subsystem of the ultrasound measurement system, in that the scan direction, tilt angle and position along the spatial axis may be controlled by the processing subsystem. It is further noted that the scan directions, tilt angles and positions shown in Figs. 3A-3D are merely exemplary.

In general, the tilt angles used in different passes may differ in sign with respect to a neutral tilt angle. For example, at least one pass may be performed using a positive tilt angle and another pass may be performed using a negative tilt angle. In some examples, the tilt angle used for both passes may be substantially identical in magnitude, in that the tilt angle may be mirror-symmetric around a neutral tilt angle. As already shown in Figs. 3A-3D, the ultrasound measurement system may acquire three or more series of images in three or more passes using three or more different tilt angles. The three or more different tilt angles may include the neutral tilt angle. Each respective tilt angle may be selected by the processing subsystem from a predetermined range, for example a range of -20 to 20 degrees or -15 to 15 degrees.

The distance over which the ultrasound transducer 200 is moved during a pass may vary depending on the anatomical structure which is to be imaged. The support structure may be designed to allow the movement of the ultrasound transducer over the desired distance. For example, for imaging of the human shoulder and elbow, it may be preferred to be able to move the ultrasound transducer over a distance of at least 4 cm. Accordingly, the support structure may be designed to allow such movement along a spatial axis. This may for example involve providing a sufficiently long guide rail. Another example is that for the patella tendon, a distance of at least 6-7 cm may be preferred. While the preceding examples were for the human body, for an animal body, different distances may need to be covered. For example, for equine tendons, movement over a distance of at least 24-30 cm may be preferred.

With continued reference to Figs. 3A-3D, which show the ultrasound transducer 200 being moved in a same direction in consecutive passes, it is noted that the ultrasound transducer 200 may also be moved along the spatial axis in alternating directions during consecutive passes. For example, the first pass may involve the ultrasound transducer 200 moving in one direction along the spatial axis 60, while in the second pass, the ultrasound transducer may move in the reverse direction. This back-and-forth movement pattern may then be repeated for any repeated passes.

With continued reference to Figs. 1A-1D, the ultrasound measurement system 100 may provide extra information which may enable a viewer of the ultrasound images, or an image processing algorithm or model, to better distinguish between specular reflections, diffuse reflections, refraction, and scattering, which in turn allows better assessment of the physical state of the imaged part of the anatomical structure.

Specular reflections: the image acquisition at different tilt angles may allow highly anisotropic structures to be better identified as they may become hypo- or even anechoic at some tilt angles.

Diffuse reflections: the image acquisition at different tilt angles may allow anisotropy to be identified, and structures having a size exceeding the ultrasound wavelength to become hypo or even anechoic (artefactually) while irregular (erratic) interface, e.g., layer of loose connective tissue (e.g., endotenon), stays reflective. Such diffuse reflections may occur when pathology is developing and may thus be better identified.

Refraction: this challenging phenomenon occurs for instance in muscle-tendon composite architecture. Structures such as fascicles (having a size exceeding the ultrasound wavelength) in muscles are hierarchically arranged under (pennate) angle to fascicles (having a size exceeding the ultrasound wavelength) in tendon. It may be impossible to insonate structures in both tendon and muscle perpendicularly under same angle of tilt; when structures in tendon are highly reflective, the structures in the muscle become artefactually hypo-reflective, and opposite. The image acquisition at different tilt angles may allow such muscle-tendon composite to be better identified and imaged. Furthermore, varying tilt angle discriminates "true" hypo-echogenicity caused by lack of structure (pathological) from "artefactual" hypo-echogenicity caused by inappropriate angle of insonation. This appears to be useful, for instance, to detect scar tissue that is not uni-axially organized.

Scattering: when tilting, the intensity of echoes (echogenicity) will not be related to angle of insonation, i.e., there may be a lack of anisotropy. The image acquisition at different tilt angles thus makes objective characterization of scattering possible.

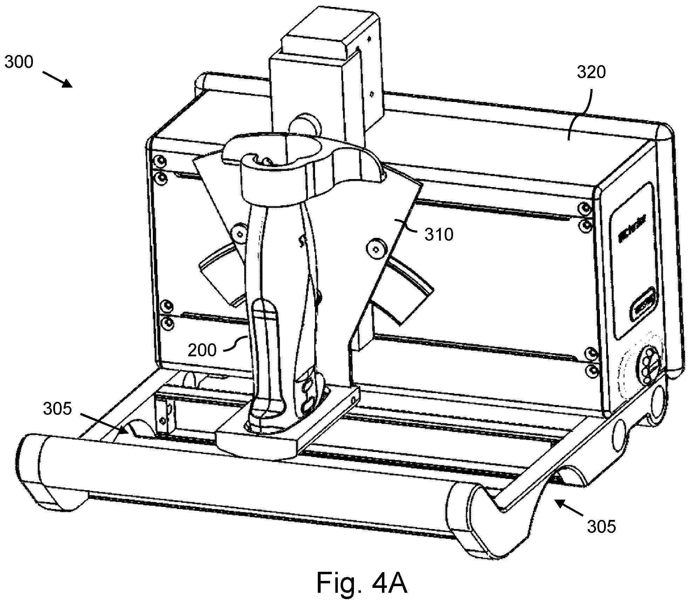

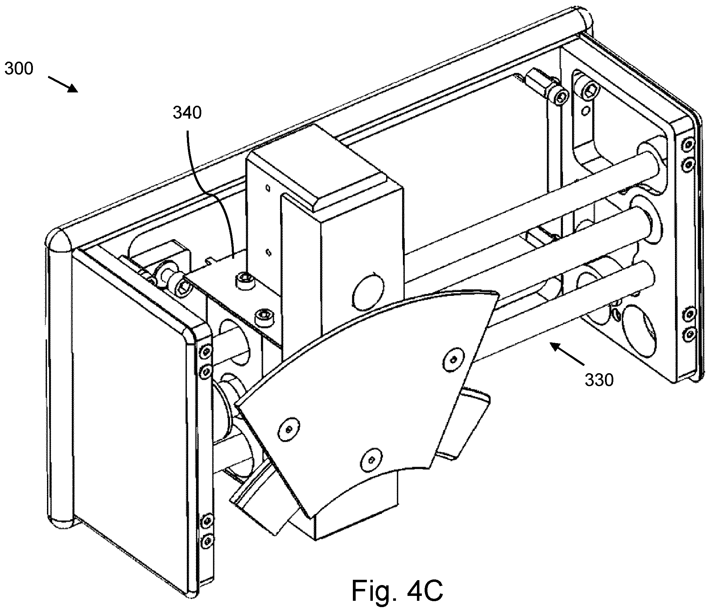

**Fig. 4A** shows an example of a support structure 300 of the ultrasound measurement system. The support structure 300 may be placed on the human or animal body. For that purpose, the support structure 300 comprise recesses 305 to accommodate a body part. This recesses are shown in Fig. 4A to be semi-circular so as to accommodate body parts having a semi-circular surface shape, such as a leg, arm, shoulder. However, this is not a limitation, in that the support structure 300 may also comprise different types of recess(es), e.g., having different shapes or dimensions or no recess at all. The support structure 300 is further shown to comprise a holding device 310 which may hold the ultrasound transducer 200. In the specific example of Fig. 4A, the holding device 310 is arranged as a mount for the ultrasound transducer 200, in that the ultrasound transducer may be mechanically mounted to the holding device. Fig. 4A further shows a housing 320 which houses electronic and mechanical components of the support structure 300, as also explained with reference to Fig. 4C.

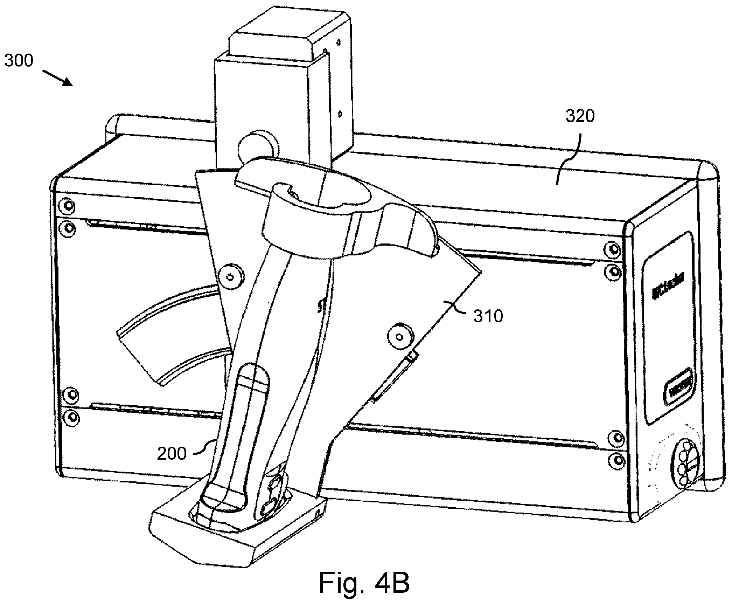

**Fig. 4B** shows the holding device 310 being tilted, which results in the tilting of the ultrasound transducer 200 about its short axis. The tilting may be actuated by one or more actuators (not shown in Fig. 4B) provided inside of the housing 320. **Fig. 4C** shows a cutaway view of the support structure 300 in which the housing is omitted. It can be seen that the support structure 300 further comprises a motorized carriage 340 to which the holding device 320 is mounted. The motorized carriage 340 may be configured to move along guide rails 330. By way of the movement of the carriage 340, the ultrasound device (not shown in Fig. 4C) may then be moved along a spatial axis, with the spatial axis being in this example the longitudinal axis of the guide rails 330.

Although not shown explicitly in Figs. 4A-4C, the support structure 300 may also comprise one or more sensors to track the movement of the motorized carriage 340 and/or the tilting of the holding device 320. Using such tracking, the ultrasound measurement system may be able to carry out the movement and tilting precisely while knowing the position and tilt angle. The sensors may for example be part of the actuators or separately provided. The support structure 300 may in general also be referred to as a 'tracker' or as a motorized tracking device' since the support structure 300 allows the ultrasound transducer 200 to perform tracked movement and tilting.

It will be appreciated that the tilting range of the ultrasound transducer 200 may be determined by the support structure 300. For example, the support structure 300 may be designed to allow tilting in a predetermined tilting range, for example in the range of [-20, 20] degrees or in the range of [-15, 15] degrees. The range may have any suitable width, and may, but does not need to be, symmetric. In a specific example the tilting range may be divided in a number of steps, e.g., 4096 steps, which may allow precise control over the tilting angle. Also the movement along the spatial axis may be precisely controlled. For example, the motorized carriage 340 may allow positioning along the spatial axis with a step size of for example 0.2 mm over a distance of for example 12 cm. However, any other values of the step size and distance over which the motorized carriage is positional are equally possible. In this respect, it is noted that if the movement and/or the tilting can be performed in a sufficiently accurate and reliable manner, there may be no need for tracking.

It will be appreciated that, in general, the support structure and its components, including the holding device, may take any other suitable form, and that the support structure 300 as shown in Figs. 4A-4C is merely exemplary.

With continued reference to the processing subsystem: the processing subsystem may be configured to control the actuation subsystem and to process the image data acquired by the ultrasound transducer. Such processing may comprise generating a visualization of the part of the anatomical structure based on the first series of images and the second series of images. For example, the processing subsystem may be configured to generate the visualization by, based on the first series of images and the second series of images, reconstructing a 3D volume showing the imaged part of the anatomical structure. Such reconstruction may be performed in any suitable manner, for example by using known 3D reconstruction techniques. As the tilt angle may be known, the tilt angle may be used as a parameter in the 3D reconstruction technique. For example, many 3D reconstruction techniques may be based on projection of image intensities of 2D images. When using such techniques, the angle of projection may be determined based on the tilt angle. Where two projection rays intersect, the 3D point of intersection may then provide a 3D voxel of the reconstructed 3D volume. The image intensity of the 3D voxel may then be determined based on the image intensities of the projected image intensities of the 2D images. This may for example involve integration or averaging to combine image intensities and/or interpolation to determine image intensities of voxels which are not intersection points. The 3D volume may then be visualized, for example by generating 2D images representing intersections of the 3D volume along sagittal, coronal, and transversal planes and by visualizing the 2D images, or by using a volume rendering technique.

In some examples, the first and second series of images may be visualized simultaneously but without combining the series of images into a single 3D volume. In other examples, in the 3D reconstruction technique, reconstructed intensities from different series of images are assigned to different colour components of the 3D volume.

In addition to control and visualization, the processing subsystem may also be configured to perform image analysis, e.g., to classify or segment the part of the anatomical structure. For example, the processing subsystem may be configured to extract features which are indicative of the presence of the anatomical structure or the part thereof. The features may be extracted from corresponding parts of the first series of images and the second series of images. As such, for a particular anatomical location, a feature vector may be obtained which comprises both a feature vector part obtained from the first series of images and a feature vector part obtained from the second series of images. In some embodiments, the feature vector may also comprise features indicative of the tilt angles. The feature vector may be used to classify and/or segment the part of the anatomical structure. In a specific example, the processing subsystem may be configured to classify and/or segment the part of the anatomical structure by using the extracted features, e.g., the extracted feature vectors, as input to a machine learned model, such as a deep neural network, or to a rule-based classifier, decision tree, random forest, gradient boosting machine, etc. In some embodiments, the visualization generated by the processing subsystem may be a visualization of the output of the image analysis. In other words, instead of, or in addition to, visualizing the 3D volume itself, an image analysis technique may be applied to the 3D volume and its output may be visualized.

The following describes a number of workflows using the ultrasound measurement system. These workflows further describe certain configurations of the ultrasound measurement system and its components, which configurations may represent embodiments of the ultrasound measurement system and its components.

A general workflow may comprise the following steps:
1. placing the B-mode ultrasound transducer at a designated location, which may involve choosing a position within three spatial dimensions, e.g., x, y, and z, and orienting the ultrasound transducer such that the pan and roll angles are set to 0 degrees, thereby fixing two of the three orientation coordinates.
2. setting the desired tilt angle φ of the ultrasound transducer.
3. moving the B-mode ultrasound transducer with a known constant speed along the designated spatial axis, while keeping the other five coordinates (two spatial and three orientation) fixed.
4. during this axial movement, capturing 2D ultrasound images captured equidistant (with step size d) intervals and storing the captured images.
5. repeating steps 2 - 4 for all the desired tilt angles φ (e.g., *T* times, for *T* different tilt values, where T is a given positive integer), where in each iteration, the direction of the movement along the designated spatial axis is reversed.

The above-described general workflow may result in the registration of T separate 3D ultrasound data sets, one for each tilt angle. As the exact location and orientation of the ultrasound transducer is known at the start of the data collection (at least substantially), and step size d and the tilt angle φ in each of the iterations are known, the T 3D ultrasound data sets may be fused into a T-dimensional ultrasound 3D voxel data set that may allow for a repeatable quantitative assessment of the ultrasonic anisotropy, 3D ultra-structures and 3D tissue characterization of the different biological and organic tissues, such as liver, ligaments, tendons, muscles, etc. Such a voxel data set may be represented as an image volume comprised of three spatial dimensions in which the voxels have T different intensity values, e.g., one for each tilt angle. The resulting voxel data set may therefore also be considered as a T+3 dimensional ultrasound data set.

The processing subsystem may execute one or more computer programs to perform the various actions described in this specification. Such computer program(s) may for example carry out the following actions, as described in pseudo-code below. In this and following examples, the support structure is with its motorized carriage and holding device referred to as 'tracker', and it is assumed that the tracker is at its home position and the scanning direction is given. Let in the following N and L be positive integers, for example N=3 and L = 600.

```
 for i=1 to N
       Let tracker tilt ultrasound transducer with angle φi
       Capture the i-th 3D ultrasound scan:
        for s=1 to L
               Capture and store B-mode ultrasound scan (e.g., of
               768x512 voxels). Let tracker displace ultrasound
               transducer with step of size d (typically d = 0.2mm)
               in designated direction.
        end for
       Change scanning direction to opposite direction.
 end for
```

Pseudocode for different types of visualization may include the following. Here, steps are distinguished with the prefix V10ZZ, with ZZ increasing from 1 to 15.

'Raw' visualization: in this example, the measurements may be visualized as-is, i.e., without rectification with respect to the tilt angle of the ultrasound transducer, and thereby without rectification with respect to the angle of scanning.

| | |
|---|---|
| V1001 | Assume N 3D ultrasound scans have been acquired with angles φ1, φ2, ..., φN, respectively. Select i from [1, N] . |
| V1002 | Select a voxel location with coordinates (x, y, z) in the i-th 3D ultrasound Scan. This may define the locations of the respective sagittal-, coronal- and transversal-planes in the i-th 3D ultrasound scan. |
| V1003 | Each plane may be visualized as a grey scale image where the intensity of the pixels may be determined by the measured ultrasound voxel at the respective location of the plane. |
| V1004 | Visualize these images, as described in V1003, in 3 panes, where pane 1, 2, and 3 are showing the sagittal-, coronal- and transversal-planes, respectively. |

Rectified visualization: in this example, the measurements may be visualized with rectification with respect to the tilt angle of the ultrasound transducer, and thereby rectified with respect to the angle of scanning.

| | |
|---|---|
| V1005 | Assume N 3D ultrasound scans have been acquired with angles φ1, φ2, ..., φN, respectively. Select i from [1, N] . |
| V1006 | Assume the i-th 3D ultrasound scan is acquired with the B-mode ultrasound transducer tilted at angle φᵢ |
| V1007 | Determine a minimal circumventing rectangular volume V in the subject that contains all the voxels of the i-th 3D ultrasound scan. |
| V1008 | Represent the voxels of V in main memory and for every voxel v of V determine the respective number(s) of the B-mode ultrasound scan(s) that intersect with voxel v and determine the location of v within the B-mode ultrasound scan(s). |
| V1009 | Assign the voxel value of the B-mode ultrasound scan(s) of the voxel that is closest to v. |
| V1010 | Visualization of the voxels of V may be done by the procedure described in V1002 - v1004. |

It is noted that in step V1009 above, a weighted interpolation of the voxels from the B-mode ultrasound scan(s) that intersect with *v* may also be used, although in some embodiments, non-interpolated values may be preferred because of the point spread function characteristics of the ultrasound beam of the B-mode ultrasound scanner and the sufficiently high precision of the scanning method using the tracker.

Integrated visualization: the N 3D ultrasound scans may be visualized using various colour encodings using Look-Up Tables (LUT's) or RGB encodings.

| | |
|---|---|
| V1011 | Assume N 3D ultrasound scans have been acquired with angles φ1, φ2, ..., φN, respectively. Assume N=3 (for other values of N a similar procedure can be followed). |
| V1012 | Determine a minimal circumventing rectangular volume V in the subject that contains all the voxels of each of the 3D ultrasound scans. |
| V1013 | For each of the N 3D ultrasound scans follow the procedure V1008 - V1009 to determine a vector of voxel values (v1, v2, ..., vN) at each voxel location in V, where vi is equal to the voxel value of the i-th 3D ultrasound scan following the procedure described in V1008 - V1009.Denote the set of voxel vectors with Ṽ. |
| V1014 | For N=3, assign the v1 value of each voxel vector of Ṽ to the R channel, assign the v2 value of each voxel vector of Ṽ to the G channel, assign the v3 value of each voxel vector of Ṽ to the B channel. |
| V1015 | The visualization of the voxel vectors of Ṽ may be done by the procedure described in V1002 - V1004, where the voxel vector intensity is depicted as RGB-pixels. (For other values of N a similar procedure can be followed.) |

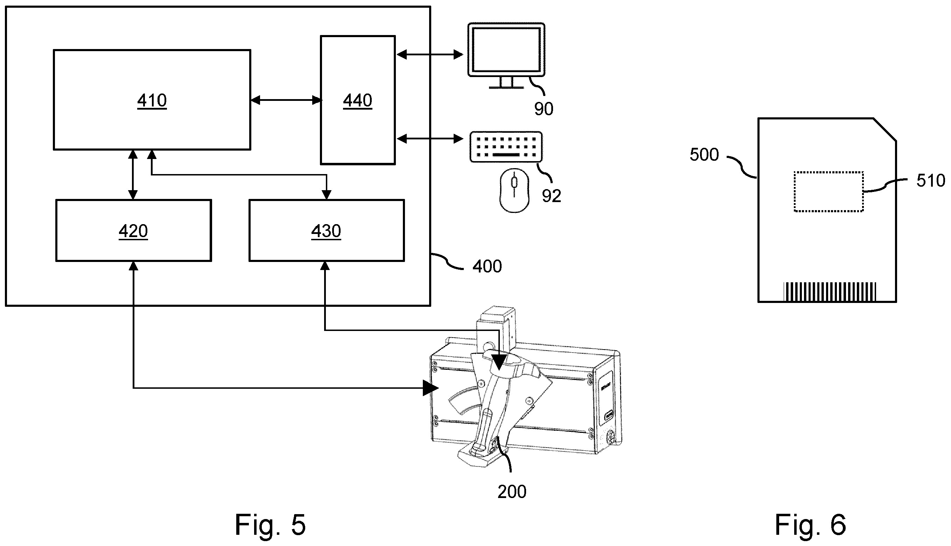

**Fig.** 5 shows an example of the processing subsystem 400 of the ultrasound measurement system in more detail. The processing subsystem 400 may comprise one or more processors 410 which may be configured to perform any of the functions described in this specification in relation to the processing subsystem. Fig. 5 shows the processing subsystem 400 to further comprise an actuator interface 420. Via the actuator interface 420, the processor(s) 410 may control the movement and tilt of the ultrasound transducer 200. The actuator interface 420 may take any suitable form, which may for example be dependent on the types of actuator(s) to be controlled. For example, the actuator interface 420 may be an electrical interface such as the Actuator Sensor Interface (ASi) or any other interface using process automation protocols or an interface using industrial control system protocols or a PC-type interface such as a Universal Serial Bus (USB) interface. The processing subsystem 400 is further shown to comprise an ultrasound transducer interface. Via the ultrasound transducer interface 430, the processor(s) may receive ultrasound data from the ultrasound transducer, which ultrasound data may be convertible into image data. In some embodiments, the ultrasound transducer interface 430 may also allow the ultrasound transducer to be configured. The ultrasound transducer interface 430 may take any suitable form, including but not limited to an electrical interface, such as a bus-type interface (e.g., USB) or a network interface or a proprietary interface for the type of ultrasound transducer, or a wireless interface (e.g., based on Wi-Fi), etc.

In some embodiments, the processing subsystem 400 may comprise a user interface subsystem 440, which user interface subsystem may be configured to, during operation of the processing subsystem 400, enable a user to interact with the processing subsystem 400, for example using a graphical user interface. For that and other purposes, the user interface subsystem 440 may comprise a user input interface (not separately shown) configured to receive user input data from one or more user input devices 92 operable by the user. The user input devices 92 may take various forms, including but not limited to a keyboard, mouse, touch screen, microphone, etc. Fig. 5 shows the user input devices to be a keyboard and mouse 92. In general, the user input interface may be of a type which corresponds to the type of user input device(s) 92, i.e., it may be a thereto corresponding type of user device interface. The user interface subsystem 440 may further comprise a display output interface (not separately shown) configured to provide display data to a display 90 to visualize output of the processing subsystem 400. Fig. 5 shows an external display 90, but alternatively, the display may be an internal display of the processing subsystem 400.

Although not shown explicitly in Fig. 5, the processing subsystem 400 may further comprise one or more communications interfaces for establishing data communication with other systems and devices. The one or more communications may include one or more wireless communication interfaces (e.g., Wi-Fi, Bluetooth, Zigbee, etc.) and/or one or more wired communication interfaces (e.g., Ethernet, optical, etc.).

In general, the processing subsystem 400 may be embodied as, or in, a single device or apparatus. The device or apparatus may be a general-purpose device or apparatus, such as a laptop or desktop computer, but may also be a dedicated device or apparatus configured to perform the functions described in this specification. The device or apparatus may comprise one or more microprocessors which may represent the processing subsystem, and which may execute appropriate software. The software may have been downloaded and/or stored in a corresponding memory, e.g., a volatile memory such as RAM or a non-volatile memory such as Flash. Alternatively, the functional units of the subsystem may be implemented in the device or apparatus in the form of programmable logic, e.g., as a Field-Programmable Gate Array (FPGA). In general, each functional unit of the processing subsystem 400 may be implemented in the form of a circuit. It is noted that the processing subsystem 400 may also be implemented in a distributed manner, e.g., involving different devices or apparatuses. For example, the distribution may be in accordance with a client-server model, e.g., using a server and workstation. For example, the user input interface and the display output interface may be part of the workstation, while the processing subsystem may be a subsystem of the server. Another example is a cloud-based implementation where the processing subsystem is cloud-based, and the user input interface and the display output interface are provided locally. It is noted that various other distributions are equally conceivable. For example, the processing subsystem may comprise a computer for high level processing and control tasks and an electronic board comprising a microprocessor for low level interfacing and processing tasks.

It will be appreciated that any method described in this specification may be implemented on a computer as a computer-implemented method, as dedicated hardware, or as a combination of both. It will be appreciated that the actions performed by the processing subsystem as described in this specification may represent examples of such a computer-implemented methods. As also illustrated in **Fig. 6**, instructions for a computer, e.g., in the form of executable code, may be stored on a computer readable medium 500, e.g., in the form of a series 510 of machine-readable physical marks and/or as a series of elements having different electrical, e.g., magnetic, or optical properties or values. The executable code may be stored in a transitory or non-transitory manner. Examples of computer readable mediums include memory devices, optical storage devices, integrated circuits, servers, online software, etc. Fig. 6 shows a memory device 500 in form of a flash memory card.

In accordance with an abstract of the present specification, a system is provided comprising a B-mode ultrasound transducer and a support structure for being placed on or near a human or animal body, wherein the support structure comprises a holding device for the ultrasound transducer which is movable and tiltable about the short axis of the ultrasound transducer. The system is configured to move the ultrasound transducer in a first pass to acquire a first series of images at a respective series of positions, move the ultrasound transducer in a second pass to acquire a second series of images at the series of positions, and tilt the holding device to have the ultrasound transducer assume first tilt angle during the first pass and a second tilt angle during the second pass. By imaging a same anatomical part using different tilting angles, it is easier to quantitatively assess the amount of anisotropy in tissue and to distinguish specular reflections and scattering.

It should be noted that the above-mentioned embodiments illustrate rather than limit the invention, and that those skilled in the art will be able to design many alternative embodiments.

In the claims, any reference signs placed between parentheses shall not be construed as limiting the claim. Use of the verb "comprise" and its conjugations does not exclude the presence of elements or steps other than those stated in a claim. The article "a" or "an" preceding an element does not exclude the presence of a plurality of such elements. Expressions such as "at least one of" when preceding a list or group of elements represent a selection of all or of any subset of elements from the list or group. For example, the expression, "at least one of A, B, and C" should be understood as including only A, only B, only C, both A and B, both A and C, both B and C, or all of A, B, and C. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

## Claims

1. An ultrasound measurement system (100), comprising:
- an ultrasound transducer (200), wherein the ultrasound transducer is a B-mode ultrasound transducer for imaging a part of an anatomical structure;
- a support structure (300) for being placed on or near a human or animal body which comprises the anatomical structure, wherein the support structure comprises a holding device (310) for the ultrasound transducer, wherein the holding device is movable along a spatial axis (60) in physical space, wherein the holding device is oriented so that a short axis (80) of the ultrasound transducer is orthogonal to the spatial axis, and wherein the holding device is tiltable about the short axis of the ultrasound transducer;
- an actuation subsystem (340) for moving the holding device and thereby the ultrasound transducer along the spatial axis and for tilting the holding device and thereby the ultrasound transducer;
- a processing subsystem (400) configured to, using the actuation subsystem and the ultrasound transducer:
- move the ultrasound transducer in a first pass along the spatial axis to acquire a first series of images at a respective series of positions along the spatial axis, move the ultrasound transducer in a second pass along the spatial axis to acquire a second series of images at the series of positions along the spatial axis, and before each pass, tilt the holding device to have the ultrasound transducer assume first tilt angle during the first pass and a second tilt angle during the second pass;
- generate a visualization of the part of the anatomical structure based on the first series of images and the second series of images.

2. The ultrasound measurement system (100) according to claim 1, wherein the first tilt angle and the second tilt angle differ in sign with respect to a neutral tilt angle.

3. The ultrasound measurement system (100) according to claim 2, wherein the first tilt angle and the second tilt angle are substantially identical in magnitude.

4. The ultrasound measurement system (100) according to any one of claims 1 to 3, wherein the processing subsystem (400) is configured to acquire three or more series of images in three or more passes using three or more different tilt angles.

5. The ultrasound measurement system (100) according to claim 4, wherein the three or more different tilt angles include a neutral tilt angle.

6. The ultrasound measurement system (100) according to any one of claims 1 to 5, wherein each respective tilt angle is selected within a range of -20 to 20 degrees, preferably within a range of -15 to 15 degrees.

7. The ultrasound measurement system (100) according to any one of claims 1 to 6, wherein the processing subsystem (400) is configured to move the ultrasound transducer in consecutive passes in alternating direction along the spatial axis.

8. The ultrasound measurement system (100) according to any one of claims 1 to 7, wherein the processing subsystem (400) is configured to generate the visualization by:
- reconstructing a 3D volume showing the imaged part of the anatomical structure based on the first series of images and the second series of images;
- visualizing the 3D volume, for example by generating 2D images representing intersections of the 3D volume along sagittal, coronal, and transversal planes and by visualizing the 2D images.

9. The ultrasound measurement system (100) according to claim 8, wherein the processing subsystem (400) is configured to generate the 3D volume using a 3D reconstruction technique and using the respective tilt angles of the ultrasound transducer as parameters in the 3D reconstruction technique.

10. The ultrasound measurement system (100) according to claim 9, wherein in the 3D reconstruction technique, reconstructed intensities from different series of images are assigned to different colour components of the 3D volume.

11. The ultrasound measurement system (100) according to any one of claims 1 to 10, wherein the processing subsystem (400) is further configured to:
- extract features pertaining to the part of the anatomical structure from corresponding parts of the first series of images and the second series of images;
- classify and/or segment the part of the anatomical structure using said extracted features.

12. The ultrasound measurement system (100) according to claim 11, wherein the processing subsystem (400) is configured to classify and/or segment the part of the anatomical structure by using the extracted features as input to a machine learned model, for example one of a deep neural network, a rule-based classifier, a decision tree, a random forest, and a gradient boosting machine.

13. The ultrasound measurement system (100) according to any one of claims 1 to 12, wherein the part of the anatomical structure is a tissue part.

14. A computer-implemented method (510) of ultrasound imaging for use with the ultrasound measurement system according to any one of claims 1 to 13, comprising, at the processing subsystem of the ultrasound measurement system:
- controlling the actuation subsystem and the ultrasound transducer to move the ultrasound transducer in a first pass along a spatial axis in physical space and to acquire a first series of images at a respective series of positions along the spatial axis;
- controlling the actuation subsystem and the ultrasound transducer to move the ultrasound transducer in a second pass along the spatial axis and to acquire a second series of images at the series of positions along the spatial axis;
- controlling the actuation subsystem to, before each pass, tilt the holding device to have the ultrasound transducer assume a first tilt angle during the first pass and a second tilt angle during the second pass; and
- generating a visualization of the part of the anatomical structure based on the first series of images and the second series of images.

15. A transitory or non-transitory computer-readable medium (500) comprising data (510) representing a computer program, the computer program comprising instructions for causing a processor system to perform the method according to claim 14.
